# EUROPEAN PATENT APPLICATION

(11) **EP 4 529 914 A1**
(43) Date of publication of application: **02.04.2025**
(21) Application number: 24202762.1
(22) Date of filing: 26.09.2024
(51) Int. Cl.: A61K 8/02, A61K 8/06, A61K 8/37, A61K 8/92, A61Q 19/00

(54) **EMULSIFYING WAX FOR SOLID COSMETIC COMPOSITIONS**

(30) Priority: 29.09.2023 IT 202300020115
(71) Applicant: Roelmi HPC S.r.l, 21040 Origgio (VA) (IT)
(72) Inventor: PADOVANO, LUIGI, 20123 Milan (IT); CONTI, Simone, 20123 MILANO (IT); PIVA, Federico, 20123 MILANO (IT)
(74) Representative: Bianchetti & Minoja with Trevisan & Cuonzo IPS SRL

(57) **Abstract**

The invention concerns an emulsifying wax, capable of incorporating water or hydrophilic substances into solid cosmetic compositions, a method for its preparation and the cosmetic products that contain it.

## Description

### Technical field of the invention

The invention relates to an emulsifying wax, capable of incorporating water or hydrophilic substances, suitable for use in the preparation of cosmetic compositions in the form of solid water/oil emulsions.

### State of the art

Solid emulsified formulations play a niche role in the cosmetics sector, in particular in the preparation of sticks and balms with a refreshing and light touch.

Formulating solid products that contain a hydrophilic phase in high concentrations is a difficult challenge for the formulator as these products are water/oil emulsions characterized by a difficult production process.

Similarly, obtaining sticks or solid cosmetic products in which it is possible to incorporate water-soluble active ingredients that are not soluble in the normal components and ingredients of an anhydrous cosmetic is an important production challenge.

The solid formulations in water/oil emulsion are made up of two phases: an oily one, for example oil and waxes which give greater viscosity to the finished product, a water/oil inverse emulsifier which allows the two phases to be stabilized and mixed, and a hydrophilic phase usually immiscible with the first.

The water/oil inverse emulsifiers most used in the cosmetics sector are usually synthetic substances based on PEG (polyethylene glycols) and PPG (polypropylene glycols), which allow the formulation of emulsion products through a conventional multi-stage process which involves the introduction of an aqueous phase in the oily phase, or vice versa, under stirring and at high temperatures, where said aqueous and oily phases are prepared separately. However, there are cases in which the use of these emulsifiers is not without problems in terms of uniformity and stability of the emulsion over time and satisfactory results are not always obtained.

WO2021239384 describes cosmetics based on water/oil emulsions, in particular lipsticks and lip creams. A composition containing sunflower seed oil (Heliantuus annuus) and polyglyceryl-3-polyricinoleate in quantities of 2.5 and 1.4% by weight respectively is exemplified. The two emulsifiers are mixed in the same phase together with several other ingredients with emulsifying properties.

There is a need to have new natural structuring and emulsifying products that guarantee uniformity and stability to the emulsions used for the preparation of cosmetic products and which also allow said emulsions to be obtained in one-pot mode, speeding up and simplifying their preparation.

### Brief description of the Figures

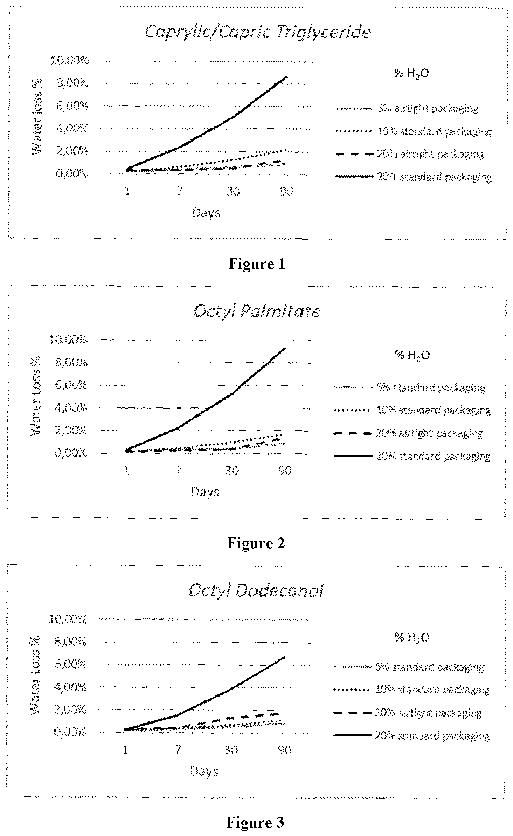

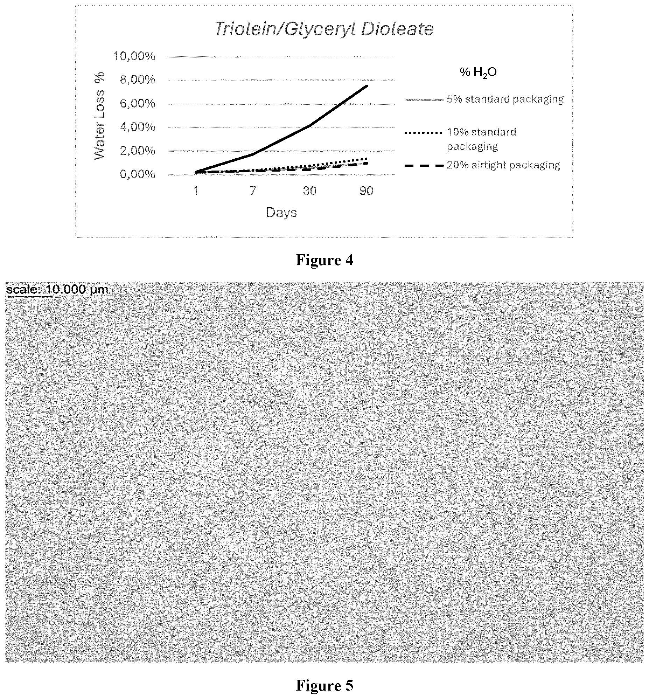

Figures 1-4 show the water loss graphs over time of solid cosmetic formulations, specifically lip balms formulated with the wax of the invention and different esters of plant origin, respectively capric/caprylic triglyceride (Figure 1), octyl palmitate (Figure 2), octyl dodecanol (Figure 3), and triolein/glyceryl dioleate (Figure 4).

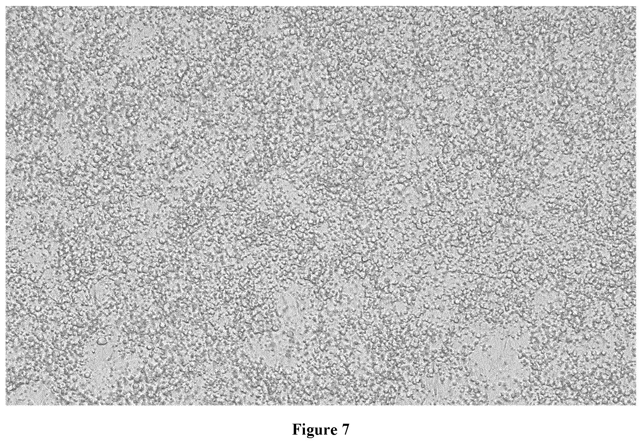

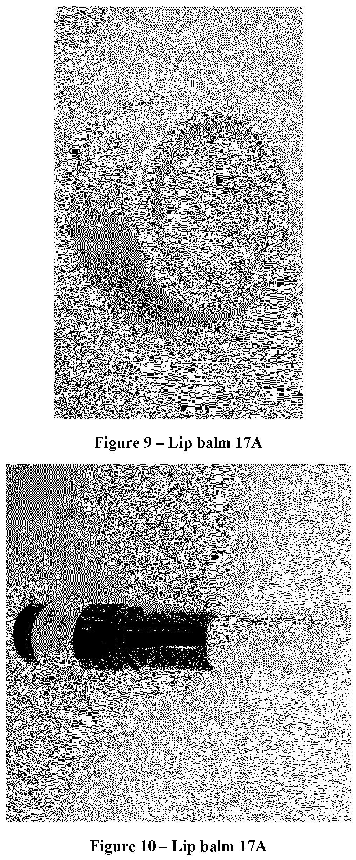

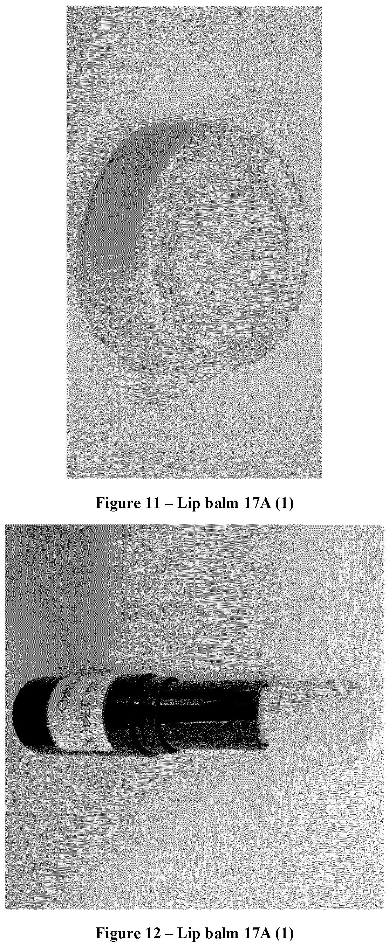

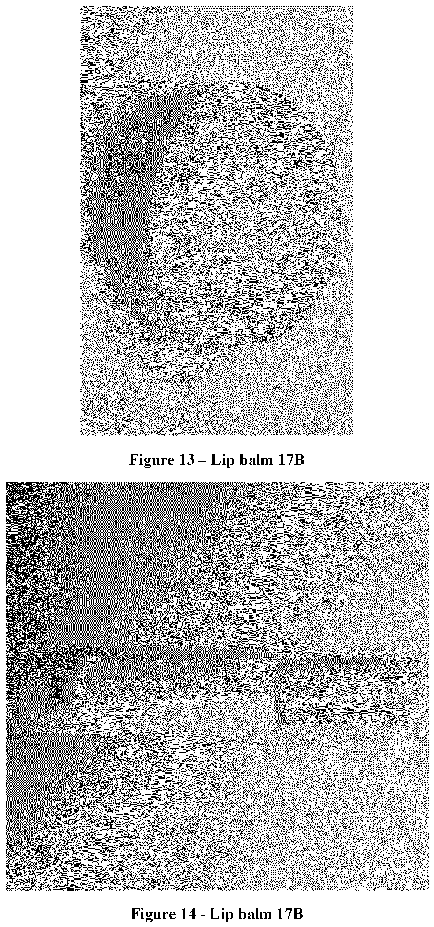

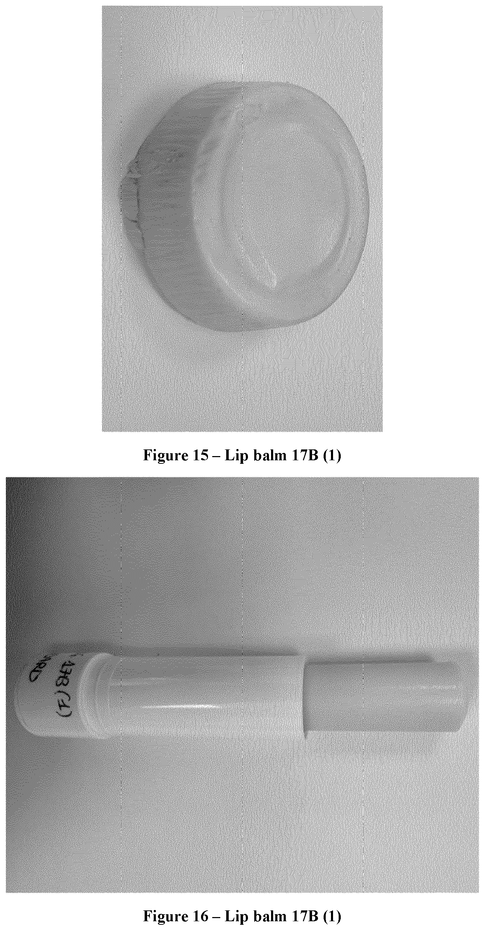

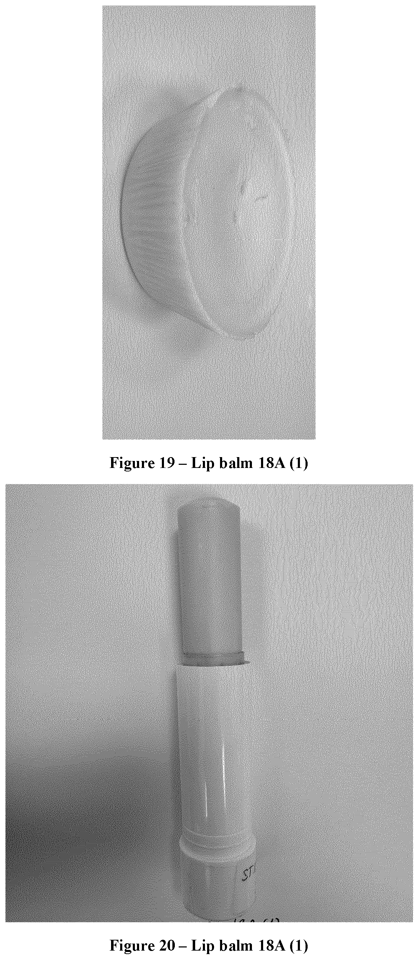

Figures 5-6 and 7-8 represent the optical microscope images of two samples of lip balm, formulated with the wax of the invention and a capric/caprylic triglyceride plant-based ester at two magnifications 50x and 100x (Figures 5 -6) and with a hydrocarbon of vegetal origin, squalane, at two magnifications 50x and 100x (Figure 7-8). Figures 9-22 describe a comparative study between some lip balms formulated using the wax of the invention (Figure 9-16) vs the separated ingredients (17-22) that composes it using diffrent production methods and different oils capric/caprylic triglyceride and octyl dodecanol.

### Detailed description of the invention

The object of the invention is an emulsifying wax consisting of:
- a component A which is sunflower seed wax (Heliantuus annuus); and
- a component B which is polyglyceryl-3-polyricinoleate or polyglyceryl-4-polyricinoleate (polyglyceryl-3/4-polyricinoleate),
wherein components A and B are in a weight ratio of 3:1 to 5:1.

It was in fact surprisingly found that by combining sunflower seed wax (Heliantuus annuus) and polyglyceryl-3/4-polyricinoleate in specific weight ratios, it was possible to obtain an emulsifying wax capable of incorporating water within a highly efficient solid matrix and to stabilize hydrophilic components that can be released on the skin and therefore particularly suitable for cosmetic use.

Furthermore, a surprising synergy was observed between the two components of the wax of the invention, respectively sunflower seed wax (Heliantuus annuus) and polyglyceryl-3/4-polyricinoleate. In the specific ratio range of the invention, emulsifying capacity, structuring and stabilizing over time and temperature was improved, as this will be evident from the experimental part.

In a preferred embodiment, the wax of the invention has the following thermal characteristics:
- Dropping point included in a range from 75 to 82°C (Analysis performed using METTLER TOLEDO Dropping point excellence System DP70/DP90 in a temperature range from 50 to 100°C with a ramp of 2°C/min); and/or
- Melting point included in a range from 71 to 80°C (DSC analysis performed via METTLER TOLEDO DSC 3 -20/130°C 10K/min N2 50mL/min); and/or
- Recrystallization point included in a range from 69 to 77°C (DSC analysis performed by METTLER TOLEDO DSC 3 DSC analysis performed by METTLER TOLEDO DSC 3 130/-20°C -10K/min N2 50mL/min).

These thermal characteristics give the wax of the invention a structuring and solidifying capacity, as well as good yield and stability when used in solid stick formulations.

The wax of the invention is a multifunctional product as, in addition to acting as an emulsifying agent with structuring properties for the lipophilic phase of the cosmetic, it is also capable of incorporating a hydrophilic phase within the wax network as a water/oil emulsion.

The hydrophilic phase is defined as a phase immiscible with non-polar substances except through the formation of an emulsion. The hydrophilic phase may contain polar and hydrophilic substances such as water, aqueous botanical/animal extracts, organic substances like glycols and polyols, alcohols, and polar esters. Additionally, it may include amino acids, proteins and peptides, carbohydrates like glucose and sucrose, urea, and inorganic salts, hydrophilic polymers, vitamins and hydrophilic acids and/or any hydrophilic active cosmetic ingredient. Furthermore, it can contain surfactants, hydrophilic lipids, and nucleic acids, which contribute to its versatility and functionality.

Structuring agents are defined as essential components for formulating products with the desired texture and stability. These agents are typically solid ingredients that enable the gelling, structuring, solidifying, or viscosity enhancement of a lipophilic phase, which usually consists of non-polar substances that are immiscible with water. By modifying the consistency and rheology of cosmetic formulations, structuring agents help create products that are stable, aesthetically pleasing, and easy to apply.

Additionally, structuring agents are used in cosmetics to create solid products such as sticks, butters, balms, and ointments. These solid formulations benefit from the structuring agents' ability to provide the necessary firmness and texture, ensuring the products are convenient to use and apply while maintaining their shape and effectiveness.

Additionally, the wax of the invention allows to formulate emulsified products both through a conventional process and in one pot mode, the latter method being particularly advantageous as it allows you to reduce production times and costs.

A further object of the invention is a method for preparing the wax of the invention comprising the steps of:
a) heat component A under stirring until it completely melts, preferably up to a temperature varying from 80°C to 100°C;
b) heat component B under stirring, preferably to a temperature varying from 60°C to 80°C;
c) mix the products obtained from steps a) and b) under stirring until a homogeneous mixture is obtained, preferably maintaining a temperature varying from 90°C to 125°C, preferably for a time varying from 15 minutes to 30 minutes;
d) cool the homogeneous mixture obtained in step c) at a temperature varying from 5°C to 15°C for a maximum time of 15 minutes, preferably up to 5 minutes; and
e) leave the mixture obtained in step d) to rest at room temperature, preferably for at least 15 minutes, until the final wax is obtained.

Advantageously, maintaining the homogeneous mixture of sunflower seeds (Helianthus annuus) and polyglyceryl-3/4-polyricinoleate, preferably at a controlled temperature ranging from 95°C to 125°C, according to step c), is carried out through a system of pipelines heated by melters during the transport to the pastillation (pastillator/piller) and/or flaking (flaker) plant.

Preferably, the homogeneous mixture is then fed, more preferably by dripping, at the aforementioned controlled temperature onto a roller rotor, the edge of which is designed with teeth of regular shape and pitch. The mixture constantly slides between the teeth; the drops accumulate on the back of each tooth, thanks to the complementary comb in contact with the toothed rotor.

In a preferred embodiment, step d) occurs through dripping from the roller rotor, where, preferably, the drops of the homogeneous mixture are deposited on a metal surface, preferably a steel belt, cooled to a temperature ranging from 5°C to 15°C by a chiller in a closed circuit, thanks to centrifugal and gravitational force, simultaneously generating the drop shape.

In this embodiment, the rotation speed of the roller rotor is synchronized with that of the steel belt to obtain a homogeneous shape of the pastilles (drops) or flakes. Step d) occurs for a maximum time of 15 minutes, preferably up to 5 minutes.

In a preferred aspect, the process of the invention may also include an additional step d') after step d), where the mixture of sunflower seeds (Helianthus annuus) and polyglyceryl-3/4-polyricinoleate is cooled by thermal shock, that is, by cooling to a temperature in the range of 5°C to 15°C within a time between 10 to 20 seconds, preferably in a pastillation (pastillator) and/or flaking (flaker) plant, always preferably by dripping.

Both steps d) and d') allow for a transformation, respectively sequential or immediate, from a liquid product to a final form that preferably appears as pearls, drops, or flakes.

A further object of the present invention is a wax obtainable by the process of the invention. The wax of the invention, preferably the wax obtainable by the process of the invention, is suitable for use in the preparation of solid cosmetic compositions in the form of sticks, butters, ointments, water/oil emulsified balms, and finds use in the field of makeup and skincare products, sunscreens, and cleansing products for the body, face, and/or hair, for example, in the form of sticks or balms, eye contour, facial and body sunscreens, lipsticks, concealers, foundations, tanning products, bronzers, contouring and blushes in stick or balm form, lip balms, and deodorants.

Therefore, a further object of the invention is the use of the wax of the invention, preferably the wax obtainable by the process of the invention, in the cosmetic field, more preferably in the sectors of makeup, sunscreens, and personal care and cleansing products.

A further object of the invention is a cosmetic product comprising the wax of the invention, preferably chosen from sticks, butters, ointments, water/oil emulsified balms, usable in the sectors of makeup and skincare, sunscreens, and cleansing products for the body, face, and/or hair, for example, in the form of sticks or balms: eye contour, facial and body sunscreens, lipsticks, concealers, foundations, tanning products, bronzers, contour and blushes in stick or balm form, lip balms, and deodorants, more preferably lip balm, lipstick, hair wax, solid BB stick, shampoo bar, tanning butter/concealer in stick form, stick deodorant, and cleansing butter.

Preferably, the wax of the invention is present in the cosmetic product in an amount of at least 5%, more preferably at least 9% of the total weight of the product. The examples reported below further illustrate the invention.

### Examples

### Materials and methods

### Example 1 - Preparation of the wax of the invention

The wax of the invention was prepared using 75 g of sunflower seeds (Heliantuus annuus) and 25 g of polyglyceryl-3-polyricinoleate, thus obtaining a weight ratio between the two of 3:1.
a) the sunflower seed wax (Heliantuus annuus) was heated under stirring to a temperature of 85°C, until the wax was completely melted;
b) the polyglyceryl-3-polyricinoleate was heated up to a temperature of 75°C, until complete melting;
c) the products obtained from steps a) and b) were mixed by maintaining a temperature of 90°C, preferably for a period of 30 minutes, until the solution was completely homogenised;
d) the homogeneous solution obtained in step c) was cooled to a temperature of 10°C, for a maximum time of 15 minutes, preferably up to 5 minutes, in order to obtain a homogeneous mixture of sunflower seed wax (Heliantuus annuus) and polyglyceryl-3-polyricinoleate;
e) the homogeneous mixture obtained in step d) was left to rest at room temperature preferably for at least 15 minutes, until 100 g of final solid wax was obtained.

### Example 2 - Preparation of a cosmetic product in solid form (lip balm) starting from the wax of the invention, according to Example 1

The following steps have been provided in Table 1.

**Table 1**

| **Phase** | **Component** | **% (w/w)** |
|---|---|---|
| A | Water | 20,00 |
| B | Wax of the invention according to Example 1 | 15,00 |
| | Caprylic/capric triglyceride | 64,90 |
| C | Phenoxyethanol | 0,10 |

a) 20 g of phase A were heated to a temperature of 85°C;
b) 79.9 g of phase B were supplied at a variable temperature of 85°C;
c) phase A was combined with phase B at a variable temperature of 85°C under turbo-stirring at 4000 rpm for 5 min;
d) during cooling, once the emulsion has taken place, 0.1 g of Phase C is added at 70°C and under stirring;
e) the emulsion is poured into a metal mold for lip balm at 65°C previously lubricated with silicone spray;
f) the metal mold with the product was left to cool at room temperature for 15min and then in the freezer for 15min at -20°C; And
g) once brought back to room temperature, the final cosmetic product was placed in a lip balm package.

The product thus composed was subsequently packaged for subsequent analyses.

The product was solid, the final emulsion stable and easily packaged.

The finished product is stable both at room temperature and at 40°C for 6 months.

### Example 3 - Preparation of a cosmetic product in solid form (lip balm) with one-pot method

The same components as Table 1 were supplied but in a single phase A:
a) phase A is heated to a temperature of 85°C, under turbo-stirring at 4000 rpm for 5 min;
b) the emulsion is poured into a metal mold for lip balm at 65°C previously lubricated with silicone spray;
c) the metal mold with the product was left to cool at room temperature for 15min and then in the freezer for 15min at -20°C; And
d) once brought back to room temperature, the final cosmetic product was placed in a lip balm package.

The product thus composed was subsequently packaged for subsequent analyses.

Even in the case of one-pot preparation, as for Example 2, the final product was solid, the final emulsion stable both at room temperature and at 40°C for 6 months and easily packaged. Furthermore, the organoleptic characteristics of the product obtained are identical to those of the lip balm prepared with the procedure according to Example 2.

### Example 4 - Comparative preparations of waxes with different components A

In order to demonstrate the unique synergy between sunflower seed oil wax (component A) and polyglyceryl-3-polyricinoleate (component B), component A was replaced by the following waxes most commonly used in the world of cosmetics, and listed in Table 2.

**Table 2**

| **Component A** | **CAS** |
|---|---|
| Cera alba or beeswax | 8012-89-3 |
| Candelilla wax or Euphorbia cerifera wax | 8006-44-8 |
| Oryza sativa rice wax | 8016-60-2 |
| Copemicia cerifera wax or carnauba wax | 8015-86-9 |

The waxes were processed following the process, conditions and quantities of Example 1, containing (as component B) polyglyceryl-3-polyricinoleate.

Subsequently, the waxes obtained were used for the preparation of lip balms, using the process of Example 2 and the one-pot process of Example 3.

The structuring and emulsifying capacity of the lip balms obtained was evaluated and the results are reported in Table 3.

**Table 3**

| **Obtained waxes** | **Weight ratios between component A:B** | **Ratings on the lip balms obtained** |
|---|---|---|
| Cera alba or beeswax + polyglyceryl-3-polyricinoleate | 3:1 | Both the multi-phase formulation and the one-pot formulation resulted in being too soft and unsuitable for a lipbalm packaging. The products obtained were not solid. |
| Candelilla wax or Euphorbia cerifera wax + polyglyceryl-3-polyricinoleate | 3:1 | Both the multi-phase formulation and the one-pot formulation resulted in being too soft and unsuitable for a lipbalm packaging. The products obtained are solids not suitable for stick cosmetic compositions. |
| Oryza sativa rice wax + polyglyceryl-3-polyricinoleate | 3:1 | Both the multi-phase formulation and the one-pot formulation resulted in being too soft and unsuitable for a lipbalm packaging. The products obtained are solids not suitable for stick cosmetic compositions. |
| Copernicia cerifera wax or carnauba wax + polyglyceryl-3-polyricinoleate | 3:1 | Both the multi-phase formulation and the one-pot formulation resulted in being liquid and unsuitable for a lipbalm packaging. |

### Example 5 - Comparative preparation of lip balm having only component A or only component B

Lip balm formulations have been developed according to Example 2 and 3:
- comparative lip balm 1) containing only component A (sunflower seed wax (Heliantuus annuus)) in the absence of component B (polyglyceryl-3-polyricinoleate), having a final composition as shown in Table 4

**Table 4**

| **Phase** | **Component** | **% (w/w)** |
|---|---|---|
| A | Water | 20,00 |
| | Component A (sunflower seed wax (Heliantuus annuus)) | 15,00 |
| | Caprylic/capric triglyceride | 64,90 |
| C | Phenoxyethanol | 0,10 |

The comparative lip balm 1) was formulated either following Example 2 or using the one-pot formulation of Example 3 and the results were the same, i.e. the final product is a nonhomogeneous and non-emulsified solid. The sunflower seed wax (Heliantuus annuus) was not able to incorporate the water, leaving it on the surface of the finished product;
- comparative lip balm 2) containing only component B (polyglyceryl-3-polyricinoleate) in the absence of component A (sunflower seed wax (Heliantuus annuus)), having a final composition as reported in Table 5.

**Table 5**

| **Phase** | **Component** | **% (w/w)** |
|---|---|---|
| | Water | 20,00 |
| B | Component B (polyglyceryl-3 polyricinoleate) | 15,00 |
| | Caprylic/capric triglyceride | 64,90 |
| C | Phenoxyethanol | 0,10 |

The comparative lip balm 2) was prepared both following Example 2 and using the one-pot formulation of Example 3 and the results were the same, i.e. the final product is a liquid emulsion which cannot be inserted into balm packaging for lips.

Furthermore, the stability of the comparative product 2) at room temperature and 40°C was followed, but after the first month there was a phase separation and the product was found to be non-stable.

This demonstrates the synergy between Component A and Component B of the wax of the invention, which has demonstrated unique properties compared to the individual ingredients that compose it.

In fact, a lip balm formulated according to Example 2 or 3, using the wax of the invention according to Example 1, was structured, emulsified and stable at room temperature and at 40°C for 6 months.

### Example 6 - comparative example in the formulation of lip balms using the wax of the invention prepared according to the production method described in the "detailed description of the invention" vs. the not premixed individual ingredients

The purpose of this example is to demonstrate the uniqueness of the production process reported in the "Detailed Description of the Invention" and then simplified in Example 1, and consequently the uniqueness of the performance of the wax of the invention obtained according to this process, compared to the use of the not premixed individual ingredients (sunflower seed wax (Helianthus annuus) and polyglyceryl-3/4 polyricinoleate) within a finished cosmetic lip balm formulation.

The formulation of the lip balms formulated using the wax of the invention produced according to the method of the "Detailed Description of the Invention" (3:1) is reported in Table 6. The same formulation was replicated with two different natural origin oils, which are caprylic/capric triglyceride or octyl dodecanol.

**Table 6**

| **Phase** | **Component** | **% (w/w)** |
|---|---|---|
| A | Water | 20,00 |
| B | Wax of the invention according to the "detailed description of the invention" | 20,00 |
| | Natural origin oil | 59,90 |
| C | Phenoxyethanol | 0,10 |

a) 20 g of phase A were heated to a temperature of 85°C;
b) 79.9 g of phase B were heated at a variable temperature of 85°C;
c) phase A was combined with phase B at a variable temperature of 85°C under turbo-stirring up to 2500 rpm for 5 min;
d) during cooling, once the emulsion has taken place, 0.1 g of Phase C is added at 70°C and under stirring;
e) the emulsion is poured into a metal mold for lip balm at 65°C previously lubricated with silicone spray;
f) the metal mold with the product was left to cool at room temperature for 15min and then in the freezer for 15min at -20°C; And
g) once brought back to room temperature, the final cosmetic product was placed in a lip balm package.

The same formulation was made using the one-pot method following the procedure below:
a) 99,9 g of every ingredient except for phenoxyethanol was heated to a temperature of 85°C;
b) at a variable temperature of 85°C the product was emulsified under turbo-stirring up to 2500 rpm for 5 min;
c) during cooling, once the emulsion has taken place, 0.1 g of phenoxyethanol is added at 70°C and under stirring;
d) the emulsion is poured into a metal mold for lip balm at 65°C previously lubricated with silicone spray;
e) the metal mold with the product was left to cool at room temperature for 15min and then in the freezer for 15min at -20°C; And
f) once brought back to room temperature, the final cosmetic product was placed in a lip balm package.

The formulation of the lip balms made using sunflower wax (Helianthus annuus) and polyglyceryl-3/4 polyricinoleate (3:1) is reported in Table 7.

**Table 7**

| **Phase** | **Component** | **% (w/w)** |
|---|---|---|
| A | Water | 20,00 |
| B | Helianthus annuus seed wax | 15,00 |
| | Polyglyceryl-3 polyricinoleate | 5,00 |
| | Natural origin oil | 59,90 |
| C | Phenoxyethanol | 0,10 |

a) 20 g of phase A were heated to a temperature of 85°C;
b) 79.9 g of phase B were heated at a variable temperature of 85°C;
c) phase A was combined with phase B at a variable temperature of 85°C under turbo-stirring up to 2500 rpm for 5 min;
d) during cooling, once the emulsion has taken place, 0.1 g of Phase C is added at 70°C and under stirring;
e) the emulsion is poured into a metal mold for lip balm at 65°C previously lubricated with silicone spray;
f) the metal mold with the product was left to cool at room temperature for 15min and then in the freezer for 15min at -20°C; And
g) once brought back to room temperature, the final cosmetic product was placed in a lip balm package.

The same formulation was made using the one-pot method following the procedure below:
a) 99,9 g of every ingredient except for phenoxyethanol was heated to a temperature of 85°C;
b) at a variable temperature of 85°C the product was emulsified under turbo-stirring up to 2500 rpm for 5 min;
c) during cooling, once the emulsion has taken place, 0.1 g of phenoxyethanol is added at 70°C and under stirring;
d) the emulsion is poured into a metal mold for lip balm at 65°C previously lubricated with silicone spray;
e) the metal mold with the product was left to cool at room temperature for 15min and then in the freezer for 15min at -20°C; And
f) once brought back to room temperature, the final cosmetic product was placed in a lip balm package.

In the attached document, images (Figures 9 - 22) of both the bulk products and the stick products are shown, where the differences between the wax of the invention and the use of the not premixed individual ingredients can be appreciated.

In Table 8, a summary of the comparative tests is provided, including the penetration analyses performed using the Penetrometer (650/SEM748, Montepaone S.R.L., San Mauro Torinese, Italy), which provides a quantitative value on the hardness/softness of the sample.

**Table 8**

| **Lip balm Name** | **Natural origin oil** | **Method** | **Penetration 1** | **Penetration 2** | **Penetration 3** | **Average Penetration** |
|---|---|---|---|---|---|---|
| 17A | Caprylic/capric triglyceride | One pot | 133 | 130 | 135 | 132,7 |
| 17A (1) | Caprylic/capric triglyceride | Standard W/O | 130 | 133 | 133 | 132,0 |
| 17B | Octyl dodecanol | One pot | 140 | 136 | 136 | 137,3 |
| 17B (1) | Octyl dodecanol | Standard W/O | 140 | 135 | 135 | 136,7 |
| 18A | Caprylic/capric triglyceride | One pot | 80 | 70 | 94 | 81,3 |
| 18A (1) | Caprylic/capric triglyceride | Standard W/O | 45 | 55 | 60 | 53,3 |
| 18B | Octyl dodecanol | One pot | NA | NA | NA | NA |
| 18B (1) | Octyl dodecanol | Standard W/O | NA | NA | NA | NA |

Where NA stands for Not Applicable, it means that the analysis was impossible to perform since the product was not homogeneous or it was not a solid that can be analyzed.

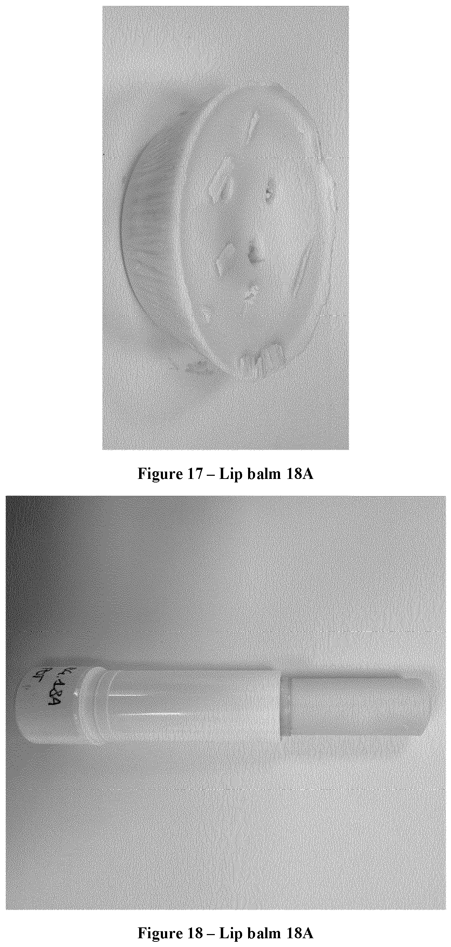

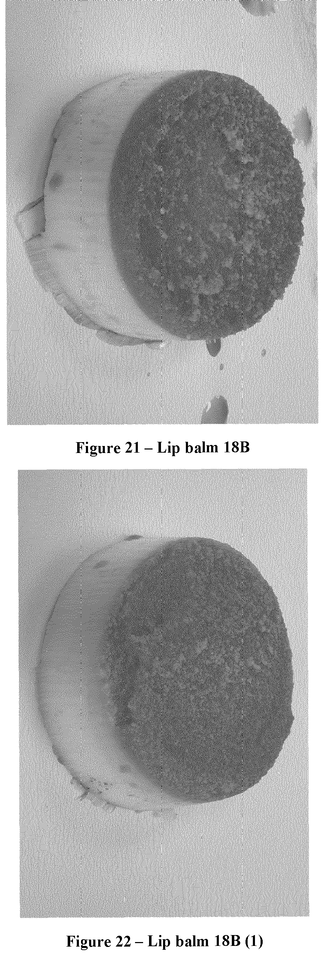

As can be seen from the results, there is a significant difference between the stick products formulated using the wax of the invention compared to the use of the not premixed individual ingredients, i.e., sunflower wax (Helianthus annuus) and polyglyceryl-3/4 polyricinoleate as if they were single ingredients in a formula. Using the wax of the invention, with both oils, the lip balms formulated in both the one-pot and standard methods produced homogeneous, structured, emulsified products that could be inserted into stick packaging. Moreover, the penetration values, repeated three times for each lip balm, were similar, producing comparable results. On the other hand, the lip balms formulated using sunflower wax (Helianthus annuus) and polyglyceryl-3/4 polyricinoleate were completely different compared to those using the wax of the invention. Observing Figures 17-20, particularly those related to sticks with caprylic/capric triglyceride (18A and 18A (1)), it is possible to note, especially in the bulk, that the product is not homogeneous and there is a separation between the emulsified phase and the waxy phase on the surface. To demonstrate this, the penetration values are lower (harder product) compared to the counterpart (17A and 17A (1)) formulated with the wax of the invention. The same can be noticed in the sticks, which are not homogeneous. For products 18B and 18B (1), it was not possible to obtain lip balms because the product was not homogeneous since it was impossibile to obtain an emulsion and could not be inserted into lip balm packaging (Figures 21-22), unlike the counterpart using the wax of the invention.

### Example 7 - Stick formulations comprising waxes with different weight ratios of A:B

Different wax compositions were tested with different ratios of component A (sunflower seed wax (Heliantuus annuus)) and component B (polyglyceryl-3-polyricinoleate), from 1:1 up to 9: 1.

The comparative waxes were used in stick formulations and each composition can be summarized as per Table 9.

**Table 9**

| **Component** | **% (w/w)** |
|---|---|
| Water | 30,00 |
| Comparative waxes (X_{A}:Y_{B}) | Z |
| Caprylic/Capric triglyceride | W |
| Phenoxyethanol | 0,10 |

Where the comparative waxes contain the ratios (X_{A}:Y_{B}), where:
- X_{A} (Component A or sunflower seed wax (Heliantuus annuus)) = 1-5 and 9
- Y_{B} (Component B or polyglyceryl-3-polyricinoleate) = 1; And
- Z (comparative wax % (w/w) relative to the total weight of the final product) = 4 - 10; And
- W (% of Caprylic/Capric Triglyceride (w/w) compared to the total weight of the final product) = 100-(Z+30+0.10).

Comparative wax compositions are summarized in Table 10.

**Table 10**

| **Z (% comparative wax (w/w))** | **(1:1)** | **(2:1)** | **(3:1)** | **(4:1)** | **(5:1)** | **(9:1)** |
|---|---|---|---|---|---|---|
| 4 | × | × | × | × | × | × |
| 5 | × | × | × | × | × | × |
| 9 | × | × | ✔ | ✔ | ✔ | ×* |
| 10 | × | × | ✔ | ✔ | ✔ | ×* |

| | | | | | | |
|---|---|---|---|---|---|---|
| Where: ✔ : indicates that, in that range of weight ratios of the A:B components in the wax, specifically from 3:1 to 5:1, the lip balm is structured, solid and emulsified, and that during the course of the six-month stability monitoring, the product has not undergone physical, chemical or organoleptic alterations. ×: indicates that, at that weight ratio of components A:B in the comparative wax, it was not possible to obtain a solid cosmetic. The final product has a consistency too soft to be placed in stick packaging. ×*: indicates that, at that weight ratio of components A:B in the comparative wax, it was not possible to obtain a homogeneously emulsified product and therefore phase separation results. | | | | | | |

Table 7 demonstrates how the best ratios between A and B are in the range of 3:1 to 5:1 and that 1:1 and 2:1 ratios at any percentage of wax used do not lead to positive results in the cosmetic product the final.

Furthermore, Table 7 demonstrates the optimal percentage of wax of the invention to use, i.e. in a concentration greater than 5%. In fact, below 5%, such as 4%, it was not possible to obtain a final solid cosmetic product.

### Example 8 - Preparations of solid cosmetic products through the use of oils of different polarity at different concentrations of water and verification of their suitability in the formulation

Various formulations for solid cosmetic products, specifically lip balms, were created by mixing the wax of the invention, obtained according to Example 1, with 0.1% (w/w) of phenoxyethanol relative to the total weight of the final product, and varying percentages of water and naturally-derived esters, using the procedures according to Examples 2 or 3.

The study focused on the ability of the wax of the invention to produce solid cosmetics using different lipid substances with varying degrees of polarity. Simultaneously, the minimum and maximum amounts of emulsifiable water that can be incorporated into the wax of the invention were evaluated.

The compositions of the obtained lip balms can be summarized as shown in Table 11.

**Table 11**

| **Phase** | **Component** | **% (w/w)** |
|---|---|---|
| A | Water | X |
| B | Wax of the invention according to Example 1 | 15,00 |
| | Ester of natural origin | Y |
| C | Phenoxyethanol | 0,10 |

| | | |
|---|---|---|
| Where: - X (percentage of water (w/w) compared to the total weight of the final product) from 5 to 30% - Y (percentage of vegetable oil (w/w) compared to the total weight of the final product) = 100- (X + 15 + 0.1) %. | | |

Different vegetable oils with varying polarity characteristics were tested, and evaluations were conducted to assess the ability of the wax of the invention, when mixed with different percentages (w/w) of water and different vegetable oils, to produce a homogeneous and stable emulsion over time at room temperature (RT) or at 40°C.

The results are reported in Table 12.

**Table 12**

| | Caprylic/Capric triglyceride (medium-high polar) | | Octyl Palmitate (mid-low polar) | | Octyl Dodecanol (medium-low polar) | | Triolein/Glyceryl Dioleate (medium polar) | |
|---|---|---|---|---|---|---|---|---|
| % water | RT stability | Stability 40°C | RT stability | Stability 40°C | RT stability | Stability 40°C | RT stability | Stability 40°C |
| 5 | ✔ | ✔ | ✔ | ✔ | ✔ | ✔ | ✔ | ✔ |
| 10 | ✔ | ✔ | ✔ | ✔ | ✔ | ✔ | ✔ | ✔ |
| 15 | ✔ | ✔ | ✔ | ✔ | ✔ | ✔ | ✔ | ✔ |
| 20 | ✔ | ✔ | ✔ | ✔ | ✔ | ✔ | ✔ | ✔ |
| 25 | × | ✔ | ✔ | ✔ | × | ✔ | ✔ | ✔ |
| 30 | × | ✔ | ✔ | ✔ | × | ✔ | ✔ | ✔ |

| | | | | | | | | |
|---|---|---|---|---|---|---|---|---|
| Where: ✔ : This indicates that, at that percentage of water and with that specific oil, the lip balm is structured, solid, and emulsified, and that during the six-month stability monitoring, the product did not undergo any physical, chemical, or organoleptic changes. ×: This indicates that, at that percentage of water and with that specific oil, the lip balm is structured, solid, and emulsified, but during the six-month stability monitoring, the product underwent surface recrystallization changes. This is a sign of instability. | | | | | | | | |

Subsequently, the lip balm formulation from Table 8, with X=20% (w/w), meaning 20% water, was tested by adding non-polar oils such as squalane (a non-polar hydrocarbon of vegetable origin) and paraffinum liquidum (a non-polar hydrocarbon of petrochemical origin), both in an amount of 64.90% (w/w) relative to the total weight of the final product.

The finished products with both oils turned out to be solid lip balm sticks that were perfectly structured, emulsified, and stable at room temperature and 40°C for 6 months.

### Example 9 - Preparations of solid cosmetic products using different concentrations of components, including water, the wax of the invention, and vegetable oils, and verification of their suitability in the formulation.

Different formulations for solid cosmetic products, specifically lip balms, were prepared by mixing 0.1% (w/w) of the total weight of the final product of phenoxyethanol with different percentages (% w/w) of water, wax of the invention, obtained according to Example 1, and vegetable oils.

The study focused on the ability of the wax of the invention to produce solid cosmetics using different lipid substances with different degrees of polarity. At the same time, the minimum and maximum quantity of emulsifiable water that can be incorporated into the wax of the invention was evaluated.

The compositions of the lip balms obtained can be summarized as per Table 13.

**Table 13**

| **Components** | **% (w/w)** |
|---|---|
| Water | X |
| Wax of the invention according to Example 1 | Y |
| Vegetable oil | Z |
| Phenoxyethanol | 0,10 |

| | |
|---|---|
| Where: - X (percentage of water (w/w) compared to the total weight of the final product) = from 7 to 20%; - Y (percentage of wax of the invention (w/w) compared to the total weight of the final product) = from 10 to 20; And - Z (percentage of esters and oils of natural origin (w/w) compared to the total weight of the final product) = 100- (X + Y + 0.1) %. | |

The following Table 14 shows the tests with respectively % Water

**Table 14**

| | **Caprylic/Capric triglyceride** | | **Octyl Palmitate** | | **Octyl Dodecanol** | | **Triolein/Glyceryl Dioleate** | |
|---|---|---|---|---|---|---|---|---|
| %Wax | RT stability | Stability 40°C | RT stability | Stability 40°C | RT stability | Stability 40°C | RT stability | Stability 40°C |
| 10 | ✔ | ✔ | ✔ | ✔ | ✔ | ✔ | ✔ | ✔ |
| 12 | ✔ | ✔ | ✔ | ✔ | ✔ | ✔ | ✔ | ✔ |
| 17 | ✔ | ✔ | ✔ | ✔ | ✔ | ✔ | ✔ | ✔ |
| 20 | ✔ | ✔ | ✔ | ✔ | ✔ | ✔ | ✔ | ✔ |

| | | | | | | | | |
|---|---|---|---|---|---|---|---|---|
| Where: ✔ : indicates that, at that percentage of water and with that specific oil, the lip balm is structured, solid and emulsified, and that during the six-month stability monitoring period the product has not undergone physical, chemical or organoleptic alterations. | | | | | | | | |

### Example 10 - Analysis of the emulsifying properties of the wax of the invention, according to Example 1 and compatibility with airtight and traditional packaging

Different formulations for solid cosmetic products, specifically lip balms, were prepared by mixing the wax of the invention, obtained according to Example 1, 0.1% (w/w) with respect to the total weight of the final product of phenoxyethanol and variable % of water and vegetable oils.

In this study, the ability of the wax of the invention to incorporate and retain increasing quantities of water within a basic lip balm formulation over time, avoiding evaporation, was evaluated.

The compositions of the lip balms obtained are shown in Table 15.

**Table 15**

| **Components** | **% (w/w)** |
|---|---|
| Water | X |
| Wax of the invention according to Example 1 | 15,00 |
| Vegetable oil | Y |
| Phenoxyethanol | 0,10 |

| | |
|---|---|
| Where: - X (percentage of water (w/w) compared to the total weight of the final product) = from 5 to 20%; - Y (percentage of vegetable oil (w/w) compared to the total weight of the final product) = 100 - (X + 15 + 0.1) %. | |

Different vegetable oils were tested, each having varying polarity characteristics, and tests were conducted to assess the ability of the wax of the invention, when mixed with different percentages (w/w) of water and different vegetable oils, to incorporate and retain increasing amounts of water over time, thus preventing evaporation, within a lip balm formulation.

The results are presented in Figures 1-4, respectively for the following vegetable oils: caprylic/capric triglyceride, octyl palmitate, octyl dodecanol, and triolein/glyceryl dioleate.

As can be observed from the Figures, for all oils, regardless of polarity, as the water concentration increases, its percentage loss over time also increases. It can also be stated that it is not necessary to use sealed packaging for volatile substances (water) up to water concentrations of 15% (w/w) relative to the total weight of the final product, as these results are consistent with those of 20% with airtight packaging. Beyond 15%, airtight packaging is necessary, which renders the loss of water over time negligible.

### Example 11 - Optical microscope analysis of cosmetic products in solid form, specifically lip balms, obtained using the wax of the invention and two different vegetable oils

Two samples were formulated according to Example 2 or Example 3, specifically:
- A lip balm 1) having the composition according to Table 16.

**Table 16**

| **Component** | **% (w/w)** |
|---|---|
| Water | 20,00 |
| Wax of the invention according to Example 1 | 15,00 |
| **Caprylic/capric triglyceride** | 64,90 |
| Phenoxyethanol | 0,10 |

- A lip balm 2) having the composition according to Table 17.

**Table 17**

| **Component** | **% (w/w)** |
|---|---|
| Water | 20,00 |
| Wax of the invention according to Example 1 | 15,00 |
| **Squalane** | 64,90 |
| Phenoxyethanol | 0,10 |

After preparation, samples were analyzed using a Nexscope NM910-TRF optical microscope in brightfield transmission mode to evaluate the ability of the wax of the invention to form microscopic micellar structures encapsulated within a solid crystalline structure.

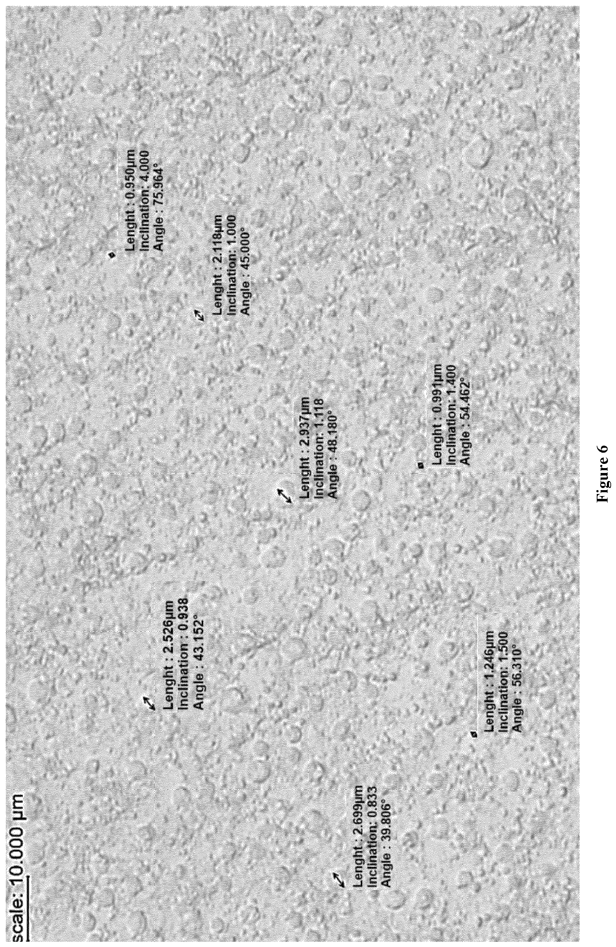

Regarding the first sample, as listed in Table 16, with caprylic/capric triglyceride as the vegetable oil, at magnifications of 50x and 100x (Figures 5 and 6), it can be observed that the surface of the lip balm is well-structured with a homogeneous distribution of micelles. Additionally, it can be inferred that the micelles are very small, with a highly uniform size distribution ranging from approximately 1µm to 3µm. Typically, such small micelles with uniform size distribution are characteristic of emulsions that are stable over time.

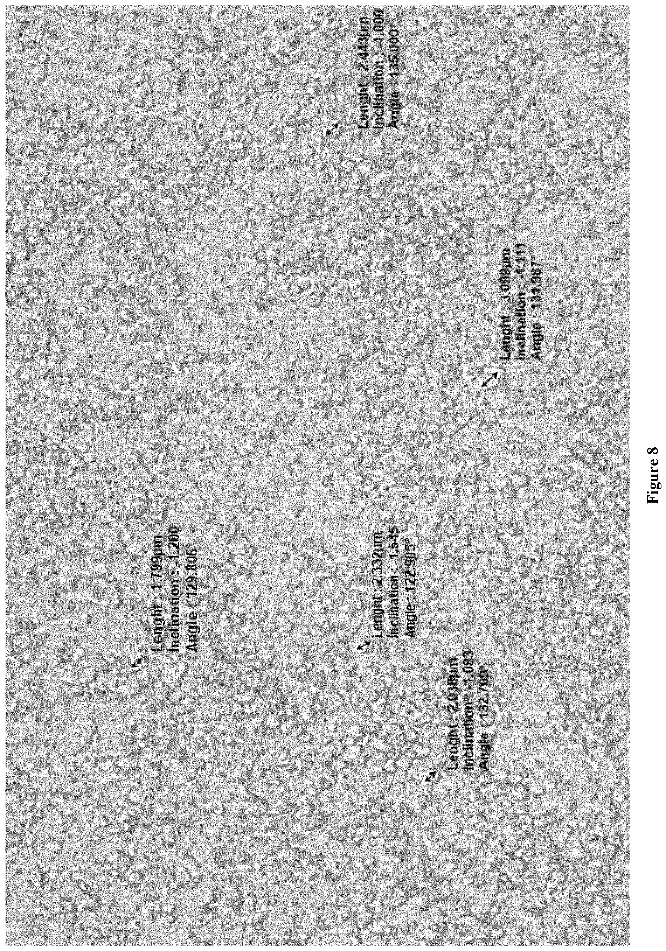

Regarding the second sample, as listed in Table 17, with squalane as the vegetable oil, at magnifications of 50x and 100x (Figures 7 and 8), it is evident that despite the lip balm being developed with a completely non-polar oil (squalane), the surface of the balm is well-structured with a homogeneous distribution of micelles. Additionally, the micelles are very small, with a uniform size distribution ranging from approximately 1.8µm to 3.1µm. Small micelles with a uniform size distribution are characteristic of emulsions that are stable over time.

In conclusion, it can be stated that the emulsions developed with the wax of the invention, regardless of the additional vegetable oil used, are of the water-in-oil (W/O) inverse type, in which water micelles are encapsulated and held in spherical form by the wax of the invention, which disperses and encapsulates them in a solid crystalline matrix.

### Example 12 - preparation of a stick lip balm according to the invention in the field of skin care

The following steps have been provided according to Table 18.

**Table 18**

| **Phase** | **Component** | **% (w/w)** |
|---|---|---|
| A | Water | 20,00 |
| | Sodium hyaluronate | 0,05 |
| | CI-14720 | 0,01 |
| | CI-42045 | 0,01 |
| B | Ethylhexyl pelargonate | 25,00 |
| | Wax of the invention according to Example 1 | 20,00 |
| | Triolein, Glyceryl Dioleate | 30,00 |
| | Ricinus communis seed oil | 2,93 |
| | Triolein, Glyceryl Dioleate, Ceramide NP | 1,50 |
| C | Phenoxyethanol | 0,1 |
| D | Perfume | 0,4 |

a) phase A was heated to a temperature of 85°C under stirring;
b) phase B was heated under stirring to a temperature of 85°C until the wax of the invention was completely melted;
c) phase A was combined with phase B at a temperature of 85°C under turbo-stirring at 4000 rpm for 5 min;
d) during cooling, once the emulsion has taken place, phase C is added at around 65-70°C and under stirring;
e) immediately afterwards phase D is added under stirring;
f) the emulsion is poured into a metal mold for lip balm at 65°C previously lubricated with silicone spray;
g) the metal mold with the product was left to cool at room temperature for 15min and then in the freezer for 15min at -20°C; And
h) once brought back to room temperature, the final cosmetic product was placed in a lip balm package.

The final product was solid, the final emulsion stable and easily packaged and stable both at room temperatures (25°C) and at 40°C for 6 months.

### Example 13 - Preparation of a lip balm according to the invention similarly to Example 12 in one-pot mode

The following steps have been provided according to Table 19.

**Table 19**

| **Phase** | **Component** | **% (w/w)** |
|---|---|---|
| A | Water | 20,00 |
| | Sodium hyaluronate | 0,05 |
| | CI-14720 | 0,01 |
| | CI-42045 | 0,01 |
| | Ethylhexyl pelargonate | 25,00 |
| | Wax of the invention according to Example 1 | 20,00 |
| | Triolein, Glyceryl Dioleate | 30,00 |
| | Ricinus communis seed oil | 2,93 |
| | Triolein, Glyceryl Dioleate, Ceramide NP | 1,50 |
| | Phenoxyethanol | 0,1 |
| | Perfume | 0,4 |

a) phase A was heated to a temperature of 85°C under stirring until the wax of the invention was completely melted;
b) phase A was emulsified at a temperature of 85°C under turbo-stirring at 4000 rpm for 5 min;
c) the emulsion is poured into a metal mold for lip balm at 65°C previously lubricated with silicone spray;
d) the metal mold with the product was left to cool at room temperature for 15min and then in the freezer for 15min at -20°C; And
e) once brought back to room temperature, the final cosmetic product was placed in a lip balm package.

The product was solid, the final emulsion stable and easily packaged. The final product was solid, the final emulsion stable and easily packaged and stable both at room temperatures (25°C) and at 40°C for 6 months. The product has the same appearance and characteristics as that obtained according to Example 12.

### Example 14 - preparation of a cleansing butter in jar according to the invention in the field of skin care

The following steps have been provided according to Table 20.

**Table 20**

| **Phase** | **Component** | **% (w/w)** |
|---|---|---|
| A | Polyglyceryl-6 Laurate | 8,00 |
| | Polyglyceryl-10 Oleate | 8,00 |
| B | Ethylhexyl Pelargonate | 30,00 |
| | Wax of the invention according to Example 1 | 12,00 |
| | *Oryza sativa bran oil* | 10,00 |
| | Tripelargonin | 26,1 |
| | Triolein, Gliceryl Dioleate, Ceramide NP | 5,00 |
| C | Tocopheryl Acetate | 0,8 |
| D | Phenoxyethanol | 0,1 |

a) phase A was heated to a temperature of 85°C under stirring;
b) phase B was heated under stirring to a temperature of 85°C until the wax of the invention was completely melted;
c) phase A was combined with phase B at a temperature of 70°C under turbo-stirring at 4000 rpm for 5 min;
d) during cooling, once the emulsion has taken place, phase C is added at 70°C and under stirring;
e) immediately afterwards phase D is added under stirring;
f) the emulsion is left to cool to room temperature until it solidifies completely.

The final product was solid, the final emulsion stable and easily packaged and stable both at room temperatures (25°C) and at 40°C for 6 months.

### Example 15 - Preparation of the cleansing butter in jar according to the invention similarly to Example 14 using one-pot method

The following steps have been provided according to Table 21.

**Table 21**

| **Phase** | **Component** | **% (w/w)** |
|---|---|---|
| A | Polyglyceryl-6 Laurate | 8,00 |
| | Polyglyceryl-10 Oleate | 8,00 |
| | Ethylhexyl Pelargonate | 30,00 |
| | Wax of the invention according to Example 1 | 12,00 |
| | *Oryza sativa bran oil* | 10,00 |
| | Tripelargonin | 26,1 |
| | Triolein, Gliceryl Dioleate, Ceramide NP | 5,00 |
| | Tocopheryl Acetate | 0,8 |
| | Phenoxyethanol | 0,1 |

a) phase A was heated to a temperature of 85°C under stirring until the wax of the invention was completely melted;
b) phase A was emulsified at a temperature of 85°C under turbo-stirring up to 4000 rpm for 5 min;
c) the emulsion is left in the cooler at room temperature until it solidifies completely.

The final product was solid, the final emulsion stable and easily packaged and stable both at room temperatures (25°C) and at 40°C for 6 months. The product has the same appearance and characteristics as that obtained according to Example 14.

### Example 16 - Preparation of solid stick deodorant according to the invention in the field of toiletries

The following steps have been provided according to Table 22.

**Table 22**

| **Phase** | **Component** | **% (w/w)** |
|---|---|---|
| A | Water | 20,00 |
| | Sodium hyaluronate | 0,1 |
| | CI 14720 | 0,01 |
| | CI 42045 | 0,01 |
| B | Ethylhexyl Pelargonate | 10,00 |
| | Wax of the invention according to Example 1 | 20,00 |
| | Triolein, Glyceryl Dioleate | 21,30 |
| | *Ricinus Communis Seed Oil* | 3,00 |
| | Triolein, Glyceryl Dioleate, Ceramide NP | 5,00 |
| | Silica | 10,00 |
| | *Zea mays corn starch, polyvinyl alcohol, glycerin* | 10,00 |
| C | Phenoxyethanol | 0,1 |
| D | Perfume | 0,48 |

a) phase A was heated to a temperature of 85°C under stirring;
b) phase B was heated under stirring to a temperature of 85°C until the wax of the invention was completely melted;
c) phase B was homogenized under turbo-stirring up to 3000 rpm for 3 min;
d) phase A and phase B were mixed at a temperature of 85°C under turbo-stirring up to 4000 rpm for 5 min;
e) during cooling, once the emulsion has taken place, phase C is added at 70°C and under stirring;
f) immediately afterwards phase D is added under stirring;
g) the emulsion is poured at 70°C into back-casting packaging for deodorants and left to cool to room temperature.

The final product was solid, the final emulsion stable and easily packaged and stable both at room temperatures (25°C) and at 40°C for 6 months.

### Example 17 - Preparation of the solid deodorant stick according to the invention similarly to Example 16 using one-pot method

The following steps have been provided according to Table 23.

**Table 23**

| **Phase** | **Component** | **% (w/w)** |
|---|---|---|
| A | Water | 20,00 |
| | Sodium hyaluronate | 0,1 |
| | CI 14720 | 0,01 |
| | CI 42045 | 0,01 |
| | Ethylhexyl Pelargonate | 10,00 |
| | Wax of the invention according to Example 1 | 20,00 |
| | Triolein, Glyceryl Dioleate | 21,30 |
| | *Ricinus Communis Seed Oil* | 3,00 |
| | Triolein, Glyceryl Dioleate, Ceramide NP | 5,00 |
| | Silica | 10,00 |
| | *Zea mays corn starch, polyvinyl alcohol, glycerin* | 10,00 |
| | Phenoxyethanol | 0,1 |
| | Perfume | 0,48 |

a) phase A was heated to a temperature of 85°C under stirring until the wax of the invention was completely melted;
b) phase A was emulsified at a temperature of 85°C under turbo-stirring at 4000 rpm for 5 min;
c) the emulsion is poured at 70°C into back-casting packaging for deodorants and left to cool to room temperature

The final product was solid, the final emulsion stable and easily packaged and stable both at room temperatures (25°C) and at 40°C for 6 months. The product has the same appearance and characteristics as that obtained according to Example 16.

### Example 18 - Preparation of a solid lipstick according to the invention in the field of makeup

The following steps have been provided according to Table 24.

**Table 24**

| **Phase** | **Component** | **% (w/w)** |
|---|---|---|
| A | Water | 20,00 |
| | Sodium hyaluronate | 0,10 |
| B | Hydrogenated Ricinus Communis Seed Oil Dimer Dilinoleate | 15,00 |
| | Wax of the invention according to Example 1 | 20,00 |
| | Pentaerythrile tetraisostearate | 19,80 |
| | Tripelargonin | 9,50 |
| | Tripelargonin, CI 15900 | 15,00 |
| C | Aroma | 0,5 |
| D | Phenoxyethanol | 0,1 |

a) phase A was heated to a temperature of 85°C under stirring;
b) phase B was heated under stirring to a temperature of 85°C until the wax of the invention was completely melted;
c) phase B was homogenized under turbo-stirring up to 6000 rpm for 10 min;
d) phase A and phase B were mixed at a temperature of 85°C under turbo-stirring at 4000 rpm for 5 min;
e) during cooling, once the emulsion has taken place, phase C is added at 70°C and under stirring;
f) immediately afterwards phase D is added under stirring;
g) the emulsion is poured at 70-75°C into a metal lipstick mold previously lubricated with silicone spray;
h) the metal mold with the product is left to cool at room temperature for 15min and then for 15min in the freezer at -20°C;
i) once brought back to room temperature, it is possible to insert the product into a lipstick package.

The final product was solid, the final emulsion stable and easily packaged and stable both at room temperatures (25°C) and at 40°C for 6 months.

### Example 19 - Preparation of the solid lipstick stick according to the invention similarly to Example 18 using one-pot method

The following steps have been provided according to Table 25.

**Table 25**

| **Phase** | **Component** | **% (w/w)** |
|---|---|---|
| A | Water | 20,00 |
| | Sodium hyaluronate | 0,10 |
| | Hydrogenated Ricinus Communis Seed Oil Dimer Dilinoleate | 15,00 |
| | Wax of the invention according to Example 1 | 20,00 |
| | Pentaerythrile tetraisostearate | 19,80 |
| | Tripelargonin | 9,50 |
| | Tripelargonin, CI 15900 | 15,00 |
| | Aroma | 0,5 |
| | Phenoxyethanol | 0,1 |

a) phase A was heated to a temperature of 85°C under stirring until the wax of the invention was completely melted;
b) phase A was emulsified at a temperature of 85°C under turbo-stirring at 5000 rpm for 6 min;
c) the emulsion is poured at 70-75°C into a metal lipstick mold previously lubricated with silicone spray;
d) the metal mold with the product is left to cool at room temperature for 15min and then for 15min in the freezer at -20°C;
e) once brought back to room temperature, it is possible to insert the product into a lipstick package.

The final product was solid, the final emulsion stable and easily packaged and stable both at room temperatures (25°C) and at 40°C for 6 months.

The final product was solid, the final emulsion stable and easily. The product has the same appearance and characteristics as that obtained according to Example 18.

### Example 20 - preparation of a contouring butter/concealer in jar according to the invention in the field of makeup

The following steps have been provided according to Table 26.

**Table 26**

| **Phase** | **Component** | **% (w/w)** |
|---|---|---|
| A | Water | 20,00 |
| | Sodium EDTA | 0,20 |
| B | Ethylhexyl Pelargonate | 19,20 |
| | *Ricinus Communis Seed Oil* | 20,00 |
| | Silica | 10,00 |
| | *Zea mays corn starch, polyvinyl alcohol, glycerin* | 10,00 |
| | CI 77891 | 5,90 |
| | CI 77491 | 1,20 |
| | CI 77499 | 0,90 |
| | CI 77492 | 2,00 |
| | Triolein, Glyceryl Dioleate, Ceramide NP | 5,00 |
| C | Wax of the invention according to Example 1 | 10,00 |
| D | Phenoxyethanol | 0,6 |

a) phase A was heated to a temperature of 85°C under stirring;
b) phase B was heated under stirring to a temperature of 85°C until the wax of the invention was completely melted;
c) phase B was homogenized at a temperature of 80°C under turbo-stirring at 6000 rpm for 10 min;
d) phase C containing the wax of the invention was added to phase B, maintaining the temperature up to 85°C until completely melted;
e) phase A was added to phases B+C at a temperature of 90°C under turbo-stirring at 4000 rpm for 5 min;
f) during cooling, once the emulsion has taken place, phase D is added at 70°C and under stirring;
g) the emulsion is poured at 70°C into a jar packaging while still liquid at a temperature of 70°C to produce a solid makeup product.

The final product was solid, the final emulsion stable and easily packaged and stable both at room temperatures (25°C) and at 40°C for 6 months.

### Example 21 - Preparation of a contouring butter/concealer in jar according to the invention similarly to Example 20 the makeup field using one-pot method

The following steps have been provided according to Table 27.

**Table 27**

| **Phase** | **Component** | **% (w/w)** |
|---|---|---|
| A | Water | 20,00 |
| | Sodium EDTA | 0,20 |
| | Ethylhexyl Pelargonate | 19,20 |
| | *Ricinus Communis Seed Oil* | 20,00 |
| | Silica | 10,00 |
| | *Zea mays corn starch, polyvinyl alcohol, glycerin* | 10,00 |
| | CI 77891 | 5,90 |
| | CI 77491 | 1,20 |
| | CI 77499 | 0,90 |
| | CI 77492 | 2,00 |
| | Triolein, Glyceryl Dioleate, Ceramide NP | 5,00 |
| | Wax of the invention according to Example 1 | 10,00 |
| | Phenoxyethanol | 0,6 |

a) phase A was heated to a temperature of 85°C under stirring until the wax of the invention was completely melted;
b) phase A was emulsified at a temperature of 90°C under turbo-stirring at 4000 rpm for 6 min;
c) the emulsion is poured at 70°C into a jar packaging while still liquid at a temperature of 70°C to produce a solid makeup product.

The final product was solid, the final emulsion stable and easily. The product has the same appearance and characteristics as that obtained according to Example 20

### Example 22 - Preparation of a balm hair wax according to the invention in the field of hair care

The following steps have been provided as per Table 28.

**Table 28**

| **Phase** | **Component** | **% (w/w)** |
|---|---|---|
| A | Water | 30,00 |
| | Cetrimonium chloride | 5,00 |
| | Pentylene glycol | 3,00 |
| | Sodium EDTA | 0,10 |
| | PVP | 7,00 |
| | Glycerin | 2,00 |
| | Butylene glycol | 2,00 |
| | Panthenol | 1,00 |
| B | Ethylhexyl Pelargonate | 11,10 |
| | Wax of the invention according to Example 1 | 14,00 |
| | Argania spinosa grain oil | 5,00 |
| | Polyglyceryl-2 isostearate/dimer dilinoleate copolymer | 5,00 |
| | Hexyl Laurate | 57,00 |
| | Zea mays corn starch, polyvinyl alcohol, glycerin | 7,00 |
| C | O-cymen-5-ol | 0,10 |
| D | Perfume | 0,70 |

a) phase A was heated to a temperature of 85°C under stirring;
b) phase B was heated to a temperature of 85°C under stirring until the wax of the invention was completely melted;
c) phase A was combined with phase B at a temperature of 85°C under turbo-stirring at 4000 rpm for 5 min;
d) during cooling, once the emulsion has taken place, phase C is added at 70°C and under stirring;
e) immediately afterwards phase D is added under stirring;
f) the emulsion is cooled with stirring to room temperature and then inserted into jar packaging.

The final product was solid, the final emulsion stable and easily packaged and stable both at room temperatures (25°C) and at 40°C for 6 months.

### Example 23 - Preparation of a balm hair wax according to the invention in the field of hair care similarly to Example 22 using one-pot method

The following steps have been provided according to Table 29.

**Table 29**

| **Phase** | **Component** | **% (w/w)** |
|---|---|---|
| A | Water | 30,00 |
| | Cetrimonium chloride | 5,00 |
| | Pentylene glycol | 3,00 |
| | Sodium EDTA | 0,10 |
| | PVP | 7,00 |
| | Glycerin | 2,00 |
| | Butylene glycol | 2,00 |
| | Panthenol | 1,00 |
| | Ethylhexyl Pelargonate | 11,10 |
| | Wax of the invention according to Example 1 | 14,00 |
| | Argania spinosa grain oil | 5,00 |
| | Polyglyceryl-2 isostearate/dimer dilinoleate copolymer | 5,00 |
| | Hexyl Laurate | 57,00 |
| | Zea mays corn starch, polyvinyl alcohol, glycerin | 7,00 |
| | o-cymen-5-ol | 0,10 |
| | Perfume | 0,70 |

a) phase A was heated to a temperature of 85°C under stirring until the wax of the invention was completely melted;
b) phase A was emulsified at a temperature of 85°C under turbo-stirring at 4000 rpm for 5 min;
c) the emulsion is cooled with stirring to room temperature and then inserted into jar packaging.

The final product was solid, the final emulsion stable and easily packaged and stable both at room temperatures (25°C) and at 40°C for 6 months.

The product has the same appearance and characteristics as that obtained according to Example 22.

### Example 24 - Preparation of a rinse-off bar balm conditioner according to the invention in the hair care field

The following steps have been provided according to Table 30.

**Table 30**

| **Phase** | **Component** | **% (w/w)** |
|---|---|---|
| A | Water | 5,00 |
| | Cetrimonium Chloride | 5,00 |
| | Polyglyceryl-6-Laurate | 5,00 |
| | Polyglyceryl-10-Oleate | 8,00 |
| | Water, Sodium Hyaluronate, Hydroxypropyl Guar, Hydroxypropyltrimonium Chloride, Pentylene Glycol, Sodium Benzoate | 2,00 |
| | Panthenol | 1,00 |
| B | Ethylhexyl Pelargonate | 10,15 |
| | Wax of the invention according to Example 1 | 16,00 |
| | Ricinus communis seed oil | 16,80 |
| | Sesamum Indicum Seed Oil | 10,00 |
| | Butyrospermum parkii butter | 5,00 |
| | Mica, CI 77891, CI 77491 | 0,05 |
| | Argania spinosa grain oil | 10,00 |
| | Zea mays corn starch, polyvinyl alcohol, glycerin | 3,00 |
| C | Caprylyl Glycol, Ethylhexylglycerin, O-cymen-5-ol | 1,00 |
| D | Perfume | 2,00 |

a) phase A was heated to a temperature of 85°C under stirring;
b) phase B was heated to a temperature of 85°C under stirring until the wax of the invention was completely melted;
c) phase A was combined with phase B at a temperature of 85°C under turbo-stirring at 4000 rpm for 5 min;
d) during cooling, once the emulsion has taken place, phase C is added at 70°C and under stirring;
e) immediately afterwards phase D is added under stirring;
f) the emulsion is poured into a silicone soap mold at a temperature of 70°C and left to cool to room temperature.

The final product was solid, the final emulsion stable and easily packaged and stable both at room temperatures (25°C) and at 40°C for 6 months.

### Example 25 - Preparation of a rinse-off bar balm conditioner according to the invention similarly to Example 24 using one-pot method in the field of haircare

The following steps have been provided according to Table 31.

**Table 31**

| **Phase** | **Component** | **% (w/w)** |
|---|---|---|
| A | Water | 5,00 |
| | Cetrimonium Chloride | 5,00 |
| | Polyglyceryl-6-Laurate | 5,00 |
| | Polyglyceryl-10-Oleate | 8,00 |
| | Water, Sodium Hyaluronate, Hydroxypropyl Guar, Hydroxypropyltrimonium Chloride, Pentylene Glycol, Sodium Benzoate | 2,00 |
| | Panthenol | 1,00 |
| | Ethylhexyl Pelargonate | 10,15 |
| | Wax of the invention according to Example 1 | 16,00 |
| | Ricinus communis seed oil | 16,80 |
| | Sesamum Indicum Seed Oil | 10,00 |
| | Butyrospermum parkii butter | 5,00 |
| | Mica, CI 77891, CI 77491 | 0,05 |
| | Argania spinosa grain oil | 10,00 |
| | Zea mays corn starch, polyvinyl alcohol, glycerin | 3,00 |
| | Caprylyl Glycol, Ethylhexylglycerin, O-cymen-5-ol | 1,00 |
| | Perfume | 2,00 |

a) phase A was heated to a temperature of 85°C under stirring until the wax of the invention was completely melted;
b) phase A was emulsified at a temperature of 85°C under turbo-stirring at 4000 rpm for 5 min;
c) the emulsion is poured into a silicone soap mold at a temperature of 0°C and left to cool to room temperature.

The final product was solid, the final emulsion stable and easily packaged and stable both at room temperatures (25°C) and at 40°C for 6 months.

The product has the same appearance and characteristics as that obtained according to Example 23.

### Example 26 - Preparation of a solid stick SPF50 BB foundation according to the invention in the field of suncare

The product is an example of a Suncare application formulation, where both inorganic and organic filters are used, furthermore, being coloured, it is a two-in-one product, suncare/make-up.

The following steps have been provided according to Table 32.

**Table 32**

| **Phase** | **Component** | **% (w/w)** |
|---|---|---|
| A | Water | 20,00 |
| | Phenylbenzimidazole Sulfonic Acid | 3,00 |
| | Water, Sodium hydroxide | Up to pH 7.1-7.2 |
| B | Ethylhexyl Pelargonate | 21,95 |
| | Wax of the invention according to Example 1 | 15,00 |
| | Diethylamino Hydroxybenzoyl Hexyl Benzoate | 5,00 |
| | Tripelargonin | 14,00 |
| | Bis-Ethylhexyloxyphenol Methoxyphenyl Triazine | 2,50 |
| | Ethylhexyl Methoxycinnamate | 3,50 |
| | Ethylhexyl Triazone | 2,00 |
| | Ethyl Silicylate | 4,00 |
| | CI 77891, Tripelargonin | 6,60 |
| | CI 77491, Tripelargonin | 0,25 |
| | CI 77499, Tripelargonin | 0,10 |
| | CI 77492, Tripelargonin | 0,80 |
| | Tocopheryl Acetate | 0,40 |
| C | Caprylyl Glycol, Ethylhexylglycerin, O-cymen-5-ol | 0,80 |

a) phase A was brought to a pH of 7.1-7.2;
b) phase A was heated to a temperature of 85°C under mechanical stirring;
c) phase B was heated to a temperature of 85°C under stirring until the wax of the invention was completely melted;
d) phase B was homogenized under turbo-stirring at 5000 rpm for 5 min;
e) phase A was combined with phase B at a temperature of 85°C under turbo-stirring at 4000 rpm for 5 min;
f) during cooling, once the emulsion has taken place, phase C is added at approximately 70°C and under stirring;
g) the emulsion of the three phases (A+B+C) is poured into a back-casting stick packaging at 70°C;
h) the packaging with the product inside is left to cool at room temperature for 15min and then for 10min in the freezer at -20°C.

The final product was solid, the final emulsion stable and easily packaged and stable both at room temperatures (25°C) and at 40°C for 6 months.

### Example 27 - Preparation of a solid stick SPF50 BB foundation according to the invention in the field of suncare similarly to Example 25 using one-pot method

The following steps have been provided according to Table 33.

**Table 33**

| **Phase** | **Component** | **% (w/w)** |
|---|---|---|
| A | Water | 20,00 |
| | Phenylbenzimidazole Sulfonic Acid | 3,00 |
| | Water, Sodium hydroxide | Up to pH 7.1-7.2 |
| B | Ethylhexyl Pelargonate | 21,95 |
| | Wax of the invention according to Example 1 | 15,00 |
| | Diethylamino Hydroxybenzoyl Hexyl Benzoate | 5,00 |
| | Tripelargonin | 14,00 |
| | Bis-Ethylhexyloxyphenol Methoxyphenyl Triazine | 2,50 |
| | Ethylhexyl Methoxycinnamate | 3,50 |
| | Ethylhexyl Triazone | 2,00 |
| | Ethyl Silicylate | 4,00 |
| | CI 77891, Tripelargonin | 6,60 |
| | CI 77491, Tripelargonin | 0,25 |
| | CI 77499, Tripelargonin | 0,10 |
| | CI 77492, Tripelargonin | 0,80 |
| | Tocopheryl Acetate | 0,50 |
| | Caprylyl Glycol, Ethylhexylglycerin, O-cymen-5-ol | 0,80 |

a) phase A was brought to a pH of 7.1-7.2;
b) phase A was heated to a temperature of 85°C under mechanical stirring;
c) the components of phase A and phase B are added directly and the mixture is heated under stirring at a temperature of 85°C until the wax is completely melted;
d) phase A was homogenized under turbo-at 4000 rpm for 5 min;
e) the emulsion is poured into a stick packaging with back filling at 70-75°C;
f) the packaging with the product inside is left to cool at room temperature for 15min and then for 10min in the freezer at -20°C.

The final product was solid, the final emulsion stable and easily packaged and stable both at room temperatures (25°C) and at 40°C for 6 months.

The product has the same appearance and characteristics as that obtained according to Example 25.

### Example 28 - Preparation of solid body exfoliating stick moisturizer according to the invention in the field of bodycare

The following steps have been provided according to Table 34.

**Table 34**

| **Phase** | **Component** | **% (w/w)** |
|---|---|---|
| A | Water | 34,00 |
| | Lactic Acid | 6,00 |
| | Salicylic Acid | 1,50 |
| | Glycerin | 3,00 |
| | Propanediol | 3,00 |
| | Water, Sodium hydroxide | Up to pH 4.2-4.5 |
| B | Octyl Palmitate | 7,00 |
| | Wax of the invention according to Example 1 | 20,00 |
| | Neopentyl Glycol Dipelargonate | 5,00 |
| | Tripelargonin | 12,40 |
| | Triolein, Glyceryl Dioleate, Ceramide NP | 3,00 |
| | Zea mays corn starch, polyvinyl alcohol, glycerin | 5,00 |
| C | Phenoxyethanol | 0,1 |

a) phase A was weighted and brought to a pH of 4.2-4.5 adding a solution of sodium hydroxide;
b) phase A was heated to a temperature of 85°C under stirring;
c) phase B was heated under stirring to a temperature of 85°C until the wax of the invention was completely melted;
d) phase B was homogenized under turbo-stirring at a velocity up to 3000 rpm for 3 min;
e) phase A and phase B were mixed at a temperature of 85°C under turbo-stirring at a velocity up to 4000 rpm for 5 min;
f) during cooling, once the emulsion has taken place, phase C is added at 70°C and under stirring;
g) immediately afterwards phase D is added under stirring;
h) the emulsion is poured at 70°C into back-filling packaging for and left to cool to room temperature.

The final product was solid, the final emulsion stable and easily packaged and stable both at room temperatures (25°C) and at 40°C for 6 months.

### Example 29 - Preparation of solid body exfoliating stick moisturizer according to the invention in the field of bodycare similarly to Example 28 using one-pot method

The following steps have been provided according to Table 35.

**Table 35**

| **Phase** | **Component** | **% (w/w)** |
|---|---|---|
| A | Water | 34,00 |
| | Lactic Acid | 6,00 |
| | Salicylic Acid | 1,50 |
| | Glycerin | 3,00 |
| | Propanediol | 3,00 |
| | Water, Sodium hydroxide | Up to pH 4.2-4.5 |
| B | Octyl Palmitate | 7,00 |
| | Wax of the invention according to Example 1 | 20,00 |
| | Neopentyl Glycol Dipelargonate | 5,00 |
| | Tripelargonin | 12,40 |
| | Triolein, Glyceryl Dioleate, Ceramide NP | 3,00 |
| | Zea mays corn starch, polyvinyl alcohol, glycerin | 5,00 |
| | Phenoxyethanol | 0,1 |

a) phase A was brought to a pH of 4.2-4.5;
b) phase B is added directly to phase A in the same container and both were heated to a temperature of 85°C under mechanical stirring;
c) the product was homogenized under turbo-at a velocity up to 4000 rpm for 5 min;
d) the emulsion is poured into a stick packaging with back filling at 70-75°C;
e) the packaging with the product inside is left to cool at room temperature for 15min and then for 10min in the freezer at -20°C.

The final product was solid, the final emulsion stable and easily packaged and stable both at room temperatures (25°C) and at 40°C for 6 months. The product has the same appearance and characteristics as that obtained according to Example 27.

## Claims

1. An emulsifying wax consisting of:
- Component A which is sunflower seed wax (Heliantuus annuus); and
- Component B which is polyglyceryl-3-polyricinoleate or polyglyceryl-4-polyricinoleate, wherein components A and B are in a weight ratio of 3:1 to 5:1.

2. A method for producing a wax consisting of:
- Component A which is sunflower seed wax (Heliantuus annuus); and
- Component B which is polyglyceryl-3-polyricinoleate or polyglyceryl-4-polyricinoleate, wherein components A and B are in a weight ratio of 3:1 to 5:1,
said method comprising the following steps:
a) Heating component A with stirring until completely melted, preferably to a temperature ranging from 80°C to 100°C;
b) Heating component B with stirring until completely melted, preferably to a temperature ranging from 60°C to 80°C;
c) Mixing with stirring the products obtained from steps a) and b) until a homogeneous mixture is obtained, preferably maintaining a temperature ranging from 90°C to 125°C, preferably for a time between 15 minutes and 30 minutes;
d) Cooling the mixture obtained in step c) to a temperature ranging from 5°C to 15°C for a maximum time of 15 minutes, preferably up to 5 minutes;
e) Allowing the mixture obtained in step d) to rest at room temperature, preferably for at least 15 minutes, until the final wax is obtained.

3. The method according to claim 2, wherein step d) is carried out by dripping, preferably onto a metal surface, more preferably at a temperature ranging from 5°C to 15°C.

4. The method according to claim 2 or 3, further comprising a step d') wherein the emulsion from step d) is cooled by thermal shock, preferably in a pastillation plant (pastillator) and/or in a flaking plant (flaker).

5. Emulsifying wax obtainable by the method according to any of claims 2-4.

6. Solid cosmetic product comprising a hydrophilic phase and a lipophilic phase, and the wax according to claims 1-5 as the sole emulsifier and/or structuring agent of said cosmetic product.

7. Solid cosmetic product according to claim 6, having a water content ranging from 1 to 40 (w/w)% preferably from 15 to 35 (w/w)%.

8. Solid cosmetic product according to claims 6-7, selected from stick, butter, ointment, balm, more preferably sunscreens, cleansing products, eye contour, lipsticks, blushes, powders, concealers, foundations, tanning/contouring/bronzing products, lip balms, deodorants, moisturizers, hair wax, solid BB stick, shampoos and conditioners bar, cleansing butter/balms.

9. A one-pot method for producing a cosmetic product according to claims 6-8 comprising mixing the hydrophilic phase, lipophilic phase, and the wax in a suitable container.
